(19)

Europäisches
Patentamt
European
Patent Office
Office européen
des brevets

(11) **EP 4 681 646 A1**

(12) **EUROPEAN PATENT APPLICATION**

(43) Date of publication:
**21.01.2026 Bulletin 2026/04**

(21) Application number: **24306190.0**

(22) Date of filing: **15.07.2024**

(51) International Patent Classification (IPC):
***A61B 6/00*** *(2024.01)*

(52) Cooperative Patent Classification (CPC):
**A61B 6/504; A61B 6/481; A61B 6/507;
A61B 6/5217; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **OLIVAN BESCOS, Javier
5656 Eindhoven (NL)**

• **HENDRIKS, Bernardus
5656 Eindhoven (NL)**
• **GRASS, Michael
5656 Eindhoven (NL)**
• **FLORENT, Raoul
5656 Eindhoven (NL)**
• **VAN MOURIK, Martijn
5656 Eindhoven (NL)**
• **ELENBAAS, Thijs
5656 Eindhoven (NL)**

(74) Representative: **Philips Intellectual Property &
Standards
High Tech Campus 52
5656 AG Eindhoven (NL)**

(54) **EVALUATING BLOOD VESSEL PROPERTIES**

(57)     A system for evaluating a property of a vessel, is provided. The system includes one or more processors that receive X-ray data representing a temporal sequence of images of the vessel. The temporal sequence of images captures the vessel over at least a portion of a cardiac cycle. The processor(s) analyze the X-ray data to evaluate at least one mechanical property of the vessel.

The at least one mechanical property of the vessel is evaluated based on a temporal variation in a cross sectional measurement $(B(x, t))$ of the vessel along a portion of the vessel and over the at least a portion of a cardiac cycle. The processor(s) output the value of the at least one mechanical property of the vessel.

**FIG. 4**

EP 4 681 646 A1

**Description**

TECHNICAL FIELD

The present disclosure relates to evaluating a property of a blood vessel. A system, a computer-implemented method, and a computer program product, are disclosed.

BACKGROUND

Blood vessel properties (e.g. geometric vessel properties, mechanical vessel properties, the composition of a deposit in the vessel, and so forth) play an important role in the assessment of vascular conditions and in the selection of their treatment options.

For instance, blood vessel properties play an important role in assessing the severity of a blood flow restriction in a vessel and in selecting a treatment option such as a Percutaneous Coronary Intervention "PCI" stenting procedure. For example, the blood vessel properties may be used to determine the blood flow in the vessel, the composition of a plaque deposit in the vessel, to assess the need to crack any calcium along the vessel wall, and also to determine the optimal position and size of the stent.

Blood vessel properties also play an important role in assessing the severity of a blood flow restriction arising from myocardial bridging. Myocardial bridging "MB" is a cardiovascular condition in which a coronary vessel is partially, or totally, encased by myocardial muscle. Although the prevalence of MB is approximately 40%, it requires treatment in only a limited number of cases. Approximately 85% of the coronary blood flow occurs during diastole, whereas MB problems occur during systolic contraction. Blood vessel properties therefore play an important role in assessing the severity of MB and in patient stratification for its treatment.

Blood vessel properties also play an important role in patient stratification for renal sympathetic denervation procedures. Renal sympathetic denervation is a procedure in which nerves around the renal artery are ablated in order to treat hypertension. However, not all patients with hypertension respond well to this type of treatment. For instance, renal sympathetic denervation has been reported as being less effective in patients with isolated systolic hypertension arising from elevated central arterial stiffness.

Although blood vessel properties can be evaluated using invasive procedures such as intravascular imaging (e.g. intravascular ultrasound "IVUS", or Optical Coherence Tomography "OCT", imaging), or by introducing other types of devices into the vasculature (e.g. a pressure wire), these options are time-consuming and cannot easily be performed prior to a treatment procedure due to their invasive nature.

Consequently, there remains a need to improve the way in which blood vessel properties are evaluated.

SUMMARY

According to one aspect of the present disclosure, a system for evaluating a property of a vessel, is provided. The system includes one or more processors configured to:

receive X-ray data representing a temporal sequence of images of the vessel, the temporal sequence of images capturing the vessel over at least a portion of a cardiac cycle;
analyze the X-ray data to evaluate at least one mechanical property of the vessel, the at least one mechanical property of the vessel being evaluated based on a temporal variation in a cross sectional measurement of the vessel along a portion of the vessel and over the at least a portion of a cardiac cycle; and
output the value of the at least one mechanical property of the vessel.

The above-described system provides the mechanical property(ies) of a vessel. The mechanical property(ies) are evaluated based on a temporal variation in a cross sectional measurement of the vessel along a portion of the vessel and over the at least a portion of a cardiac cycle. The inventors have found that mechanical property(ies) for the vessel may be determined by evaluating the temporal, i.e. dynamic, behavior of the portion of the vessel in this manner. The mechanical property(ies) are specific to the vessel and may be used to assess the severity of a vascular condition and to select a treatment option. Moreover, since the system provides the mechanical property(ies) from X-ray data, it obviates the need for an invasive procedure such as intravascular imaging, or the introduction of a pressure wire into the vessel.

Further aspects, features, and advantages of the present disclosure will become apparent from the following description of examples, which is made with reference to the accompanying drawings.

BRIEF DESCRIPTION OF THE DRAWINGS

Fig. 1 is a schematic diagram illustrating an example of a system 100 for evaluating a property of a vessel, in accordance with some aspects of the present disclosure.

Fig. 2 is a flowchart illustrating an example of a computer-implemented method of evaluating a property of a vessel, in accordance with some aspects of the present disclosure.

Fig. 3 is a schematic diagram illustrating an example of a vessel deformation pulse 150 propagating along a vessel, in accordance with some aspects of the present disclosure.

Fig. 4 is a schematic diagram illustrating, in a different manner to Fig. 3, an example of a vessel deformation pulse 150 propagating along a vessel, in accordance with some aspects of the present disclosure.

Fig. 5 is a schematic diagram illustrating an example of a technique for determining a temporal velocity profile $VCA_2(t)$ of a contrast agent along a vessel 130, in accordance with some aspects of the present disclosure.

Fig. 6 is a flowchart illustrating an example of a method of evaluating a haemodynamic parameter for a vessel based on a value of a mechanical property of the vessel, in accordance with some aspects of the present disclosure.

Fig. 7 is a flowchart illustrating an example of a method of evaluating a myocardial bridging metric for a vessel based on a value of a mechanical property of the vessel, in accordance with some aspects of the present disclosure.

DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system for evaluating a property of a vessel. In some examples, reference is made to a vessel in the form of coronary artery. It is, however, to be appreciated that the artery, and the heart, serve only as examples. In general, the system may be used to evaluate a property of any type of blood vessel, and the blood vessel may be in any anatomical region. For instance, the system may be used to evaluate a property of an artery, or a vein, and the blood vessel may be in the heart, the brain, or in a peripheral region such as the leg, the kidney, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

It is also noted that some operations that are described as being performed by one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need to improve the way in which blood vessel properties are evaluated.

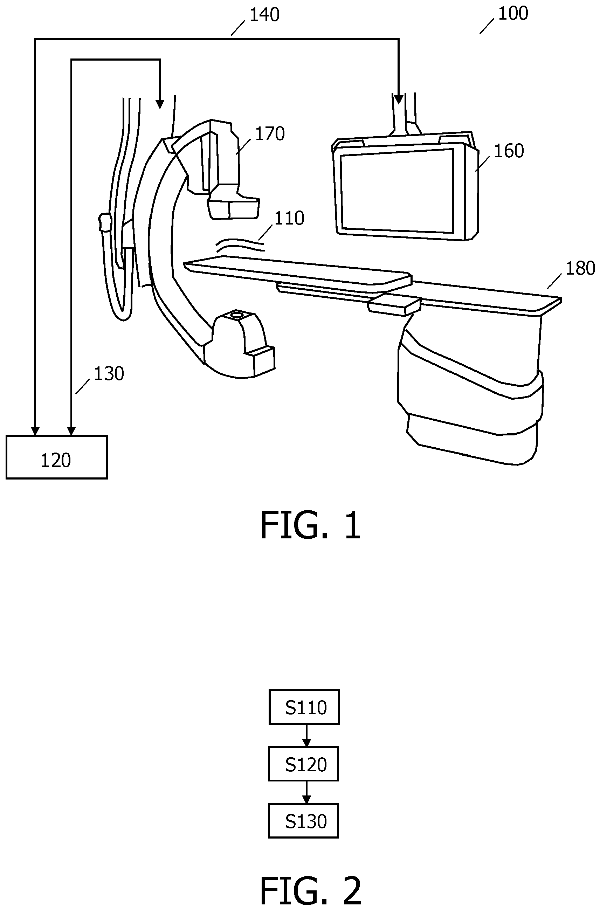

Fig. 1 is a schematic diagram illustrating an example of a system 100 for evaluating a property of a vessel, in accordance with some aspects of the present disclosure. Fig. 2 is a flowchart illustrating an example of a computer-implemented method of evaluating a property of a vessel, in accordance with some aspects of the present disclosure. It is noted that operations that are described as being performed by the one or more processors 120 of the system 100 illustrated in Fig. 1, may also be performed in the method illustrated in Fig. 2. Likewise, operations that are described in relation to the method described with reference to Fig. 2 may also be performed by the one or more processors 120 of the system 100 illustrated in Fig. 1. With reference to Fig. 1, and Fig. 2, the system 100 for evaluating a property of a vessel 110, includes one or more processors 120 configured to:

receive S110 X-ray data 130 representing a temporal sequence of images of the vessel 110, the temporal sequence of images capturing the vessel 110 over at least a portion of a cardiac cycle;

analyze S120 the X-ray data 130 to evaluate at least one mechanical property 140 of the vessel 110, the at least one mechanical property of the vessel being evaluated based on a temporal variation in a cross sectional measurement $B(x, t)$ of the vessel along a portion of the vessel and over the at least a portion of a cardiac cycle; and

output S130 the value of the at least one mechanical property 140 of the vessel 110.

The above-described system 100 provides the mechanical property(ies) of a vessel. The mechanical property(ies) are evaluated based on a temporal variation in a cross sectional measurement of the vessel along a portion of the vessel and over the at least a portion of a cardiac cycle. The inventors have found that mechanical property(ies) for the vessel may be determined by evaluating the temporal, i.e. dynamic, behavior of the portion of the vessel in this manner. The mechanical property(ies) are specific to the vessel and may be used to assess the severity of a vascular condition and to select a treatment option. Moreover, since the system provides the mechanical property(ies) from X-ray data, it obviates the need for an invasive procedure such as intravascular imaging, or the introduction of a pressure wire into the vessel.

The operations that are performed by the processor(s) 120 of the system 100 are described in more detail below.

Referring initially to the operation S110 illustrated in Fig. 2; in this operation, X-ray data 130 is received. The X-ray data 130 represents a temporal sequence of images of the vessel 110.

The X-ray data 130 that is received in the operation S110 may be generated by various types of X-ray imaging systems. In general, these include two-dimensional "2D" X-ray imaging systems, i.e. projection X-ray imaging systems, and also three-dimensional "3D", or "volumetric", X-ray imaging systems, i.e. computed tomography "CT" imaging systems. Projection images may be generated from X-ray data that is generated by a projection imaging system, and volumetric images may be generated from X-ray data acquired from a CT imaging system. Thus, in one example, the X-ray data 130 represents a temporal sequence of projection images of the vessel 110. In another example the X-ray data 130 represents a temporal sequence of volumetric images of the vessel 110. In one example, the X-ray data 130 represents a live temporal sequence of images of the vessel 110. In another example, the X-ray data 130 represents a previously-recorded temporal sequence of images.

As mentioned above, in general, CT imaging systems may be used to generate volumetric, i.e. CT, images. CT images include depth information relating to an anatomical structure. By contrast, projection X-ray imaging systems are typically limited to providing projection X-ray images of an anatomical structure. Due to their projected nature, projection X-ray images lack accurate depth information. However, depth information may be estimated from projection images that are acquired by a projection X-ray imaging system from multiple different viewing angles with respect to an anatomical structure. The same applies to temporal sequences of projection X-ray images. Thus, in one example, the X-ray data 130 that is received in the operation S110 represents a temporal sequence of projection images of the vessel 110 from each of a plurality of different viewing angles with respect to the vessel. The images in the temporal sequences from the different viewing angles may be acquired substantially simultaneously. As described in more detail below, the images from the different viewing angles provide information relating to the temporal variation in cross sectional measurements of the vessel in different directions. In-turn, this facilitates the evaluation of a more accurate mechanical property(ies) of the vessel.

In some examples, the X-ray data 130 that is received in the operation S110 is generated by a fluoroscopic imaging system. A fluoroscopic imaging system refers generally to an imaging device that uses X-rays to generate a temporal sequence of images. The temporal sequence of images may be referred-to as fluoroscopic images. An X-ray dose that is used to acquire a fluoroscopic image may be relatively lower than a dose used to acquire a conventional X-ray image. Both volumetric and projection fluoroscopic imaging systems are known. An example of a fluoroscopic imaging system is the Azurion 5 system marketed by Philips Healthcare, Best, The Netherlands.

In general, the X-ray data 130 may be generated by a conventional X-ray imaging system or by a spectral X-ray imaging system. The X-ray imaging system that generates the X-ray data 130 may therefore be a projection spectral X-ray imaging system, or a volumetric spectral X-ray imaging, i.e. a spectral CT imaging system. Such spectral X-ray imaging systems generate X-ray data representing X-ray attenuation within multiple different energy intervals. The X-ray data generated by such spectral imaging systems may be processed, e.g. using various material decomposition algorithms, in order to distinguish between media, such as contrast agent, tissue, bone, the constituents of deposits in vessels such as plaque, and so forth, and which have similar X-ray attenuation values when measured within a single energy interval, and which would be indistinguishable in X-ray data generated by conventional X-ray imaging systems.

In some examples, the X-ray data 130 that is received in the operation S110 is generated subsequent to the injection of a contrast agent into the vasculature of a subject. The contrast agent serves to improve the contrast between blood within a vessel and tissue surrounding the vessel in the resulting temporal sequence of images. In these examples, the temporal sequence of images of the vessel 110 may be referred-to as a temporal sequence of contrast-enhanced images of the vessel.

With continued reference to the operation S110 illustrated in Fig. 2; the X-ray data 130 that is received in this operation represents a temporal sequence of images capturing the vessel 110 over at least a portion of a cardiac cycle. As mentioned above, the vessel may be a coronary artery, or it may be another vessel. By way of an example, the vessel may be the left anterior descending "LAD" coronary artery. The X-ray data 130 may capture the vessel 110 over at least the diastole phase of the cardiac cycle, or at least the systole phase of the cardiac cycle, or at least another portion of the cardiac cycle. For instance, the X-ray data 130 may capture the vessel 110 over a complete cardiac cycle, or over a duration that is longer than a complete cardiac cycle.

With continued reference to the operation S110 illustrated in Fig. 2; the X-ray data 130 that is received in this operation may be received from various sources. For instance, the X-ray data 130 may be received from an X-ray imaging system, such as from one of the X-ray imaging systems described above. The X-ray data 130 may alternatively be received from a different source, such as from a computer-readable storage medium, of from the Internet, or the Cloud, and so forth. The X-ray data 130 may be received via any form of data communication in the operation S110, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S 120 illustrated in Fig. 2; in this operation, the X-ray data 130 is analyzed in order to evaluate at least one mechanical property 140 of the vessel 110. Changes in the pressure within blood vessels over the cardiac cycle give rise to pulsatile variations in their cross sectional measurements. For instance, changes in the pressure within a coronary artery over the cardiac cycle give rise to approximately a 5 - 10 percent variation in its diameter. For a coronary artery with a diameter of approximately three millimeters, this corresponds to a variation in its diameter of 0.15 - 0.3 millimeters. The inventors have observed that variations of this magnitude may be detected in X-ray images. Moreover, because the pulsatile variations in the cross sectional measurements of a vessel are affected by its mechanical properties (e.g. its stiffness), the mechanical properties of the vessel may be determined by analyzing a temporal sequence of images that captures changes in cross-sectional measurements (e.g. the diameter) of the vessel during the cardiac cycle.

Examples describing the analysis of the temporal sequence of images in the operation S 120 are described in detail below. These include an example in which mechanical property(ies) 140 of the vessel 110 are evaluated by determining a velocity of a vessel deformation pulse 150 propagating along the vessel 110, and another example in which mechanical property(ies) 140 of the vessel 110 are evaluated by inputting a temporal sequence of images of the vessel into a trained machine learning algorithm.

Various mechanical properties of the vessel may be evaluated in the operation S 120. These include a stiffness of the vessel, and also other mechanical properties, such as the Young's modulus, or the elasticity, or the shear stress, for example. Such properties affect the pulsatile variations in the cross sectional measurements of a vessel during the cardiac cycle. The mechanical property(ies) 140 may be evaluated based on a temporal variation in a various cross sectional measurements of the vessel. For instance, the mechanical property 140 may be evaluated based on a temporal variation in a diameter, or a radius, or a cross-sectional area, or another cross sectional measurement, of the vessel. The mechanical property(ies) 140 of the vessel 110 may be evaluated based on a temporal variation in the cross sectional measurement of the vessel along any desired portion of the vessel. For instance the mechanical property(ies) 140 may be evaluated based on a temporal variation in the cross sectional measurement of the vessel along a portion of the vessel between two bifurcations, or along a portion of the vessel between a point marking a proximal end of the vessel and a point marking a distal of the vessel.

Referring now to the operation S130; in this operation, the value of the at least one mechanical property 140 of the vessel 110 is outputted. The value of the at least one mechanical property 140 of the vessel 110 may be outputted in various ways. For instance, it may be outputted to a display device, such as to the monitor 160 illustrated in Fig. 1, or to a virtual/ augmented reality display device, or to printer, or to a computer-readable storage medium, and so forth.

Further examples of the system 100 are described in the examples below.

In one example, the X-ray data 130 represents a live temporal sequence of images of the vessel 110. In this example, the operation of analyzing S120 the X-ray data, and the operation of outputting S130 the value of the at least one mechanical property 140 of the vessel 110, are performed for a current image in the live sequence and repeated for subsequent current images in the live sequence.

In this example, the system 100 may be used to provide the mechanical property(ies) of a vessel substantially in real-time.

In one example, the one or more processors 120 are configured to evaluate a quality metric corresponding to the value of the at least one mechanical property 140 of the vessel 110, and to output an indication of the quality metric.

In this example the quality metric provides confidence to a user of the system in the evaluated mechanical property(ies). The quality metric may be evaluated based on factors such as an image quality of the images in the temporal sequence, or a quality (e.g. a signal to noise ratio, or a variation over time) of a pulsation signal calculated by tracking the vessel deformation over time in the images in the temporal sequence, for example.

In one example, the one or more processors 120 are configured to evaluate, based on the value of the at least one mechanical property 140 of the vessel 110, a suitability of one or more treatment procedures for the vessel. In this example, the one or more processors are also configured to output an indication of the suitability of the one or more treatment procedures.

In this example, the suitability may for instance be a suitability of a renal sympathetic denervation procedure, or a suitability of a stenting procedure for instance. As described above, renal sympathetic denervation have been reported as being less effective in patients with isolated systolic hypertension arising from elevated central arterial stiffness. Thus, if the value of the stiffness of the vessel exceeds a threshold value, it may be recommended that a subject is excluded from a renal sympathetic denervation treatment procedure. The suitability may therefore be evaluated by applying a threshold condition to the value of the at least one mechanical property 140 of the vessel 110.

Referring now in more detail to the operation S120 in which the X-ray data is analyzed. In one example, the operation of analyzing the X-ray data 130 includes determining, based on the temporal variation in the cross sectional measurement $B(x, t)$ of the vessel at each of a plurality of positions, $x_0$, $x_2$, along the portion of the vessel and over the at least a portion of a cardiac cycle, a velocity $v(x_0, T_0)$, $v(x_2, T_2)$, $v_{average}(x_0{\rightarrow}x_2, T_0{\rightarrow}T_2)$ of a vessel deformation pulse 150 propagating along the vessel 110. In this example, the evaluation of the at least one mechanical property 140 is performed based on the velocity $v(x_0, T_0)$, $v(x_2, T_2)$, $v_{average}(x_0{\rightarrow}x_2, T_0{\rightarrow}T_2)$ of the vessel deformation pulse 150.

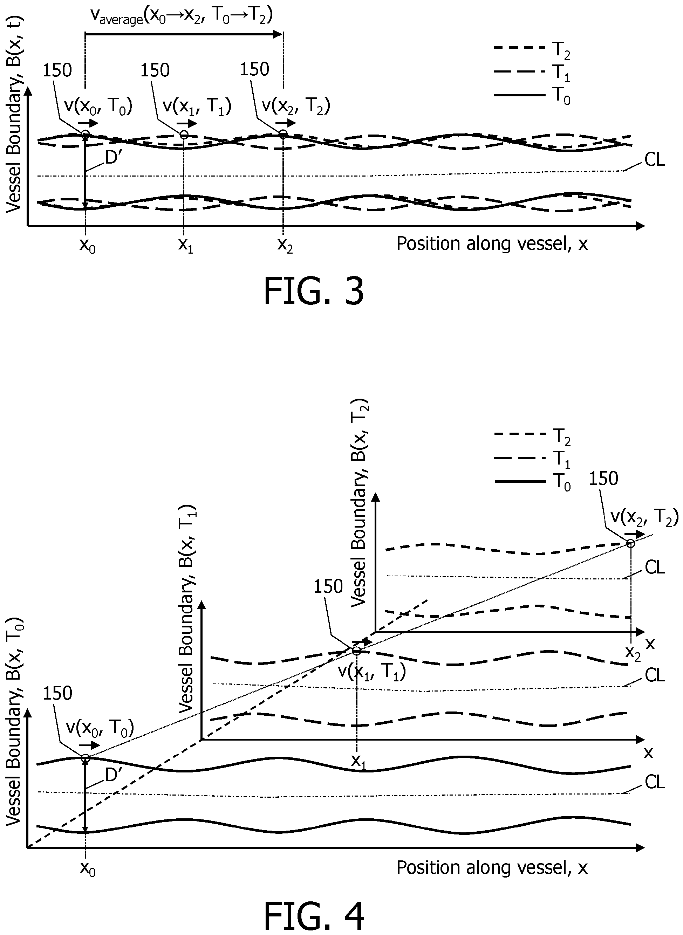

This example is described with reference to Fig. 3, and Fig. 4. Fig. 3 is a schematic diagram illustrating an example of a vessel deformation pulse 150 propagating along a vessel, in accordance with some aspects of the present disclosure. Fig. 3 includes a graph representing the diameter of a vessel on the ordinate axis against position, x, along the vessel on the abscissa, at each of times $T_0$, $T_1$, and $T_2$. In the illustrated example, the diameter of the vessel is represented by the separation, measured on the ordinate axis, between the corresponding upper and lower boundary of the vessel, and which are illustrated respectively via two corresponding lines, at each of times $T_0$, $T_1$, and $T_2$. For instance, at time $T_0$, the solid lines in Fig. 3 illustrate the corresponding upper and lower boundary of the vessel at each of multiple positions, x, along the vessel. At time $T_0$, the separation D' between the upper and lower boundary of the vessel represents the projected diameter of the vessel. The long-dashed lines in Fig. 3 illustrate the corresponding upper and lower boundary of the vessel at each of multiple positions, x, along the vessel at time $T_1$. Similarly, the short-dashed lines in Fig. 3 illustrate the corresponding upper and lower boundary of the vessel at each of multiple positions, x, along the vessel at time $T_2$.

Fig. 4 is a schematic diagram illustrating, in a different manner to Fig. 3, an example of a vessel deformation pulse 150 propagating along a vessel, in accordance with some aspects of the present disclosure. Fig. 4 illustrates the same information as that illustrated in Fig. 3. However, in Fig. 4, the graphs representing the diameter of the vessel are shown separately for each of times $T_0$, $T_1$ and $T_2$, for ease of visualization.

The graphs illustrated in Fig 3 and in Fig. 4 may be obtained from the temporal sequence of images of the vessel 110 using known image processing techniques. For instance, the upper and lower boundary of the vessel illustrated in Fig. 3 may be determined by segmenting the vessel in the temporal sequence of images that is received in the operation S110, in order to identify the vessel lumen. Known image segmentation techniques may be used for this purpose. The extent of the vessel lumen in projected images defines the corresponding upper and lower boundary of the vessel illustrated in Fig. 3 and in Fig. 4.

In some examples, the images in the temporal sequence may be mutually registered, e.g. based on a reference in the vessel in the images, such as a landmark (e.g. a vessel bifurcation, or a calcium deposit), the vessel centerline, CL, and so forth, prior to determining the upper and lower vessel boundary of the vessel illustrated in Fig. 3 and in Fig. 4. Mutually registering the images provides a more accurate delineation of the vessel boundary, particularly in the presence of (cardiac) vessel motion, and consequently improves the accuracy of cross-sectional measurements of the vessel that are analyzed in the operation S120.

As may be seen from the graphs illustrated in Fig. 3 and Fig. 4, at each of times $T_0$, $T_1$ and $T_2$, there is a variation in the diameter of the vessel with position, x, along the vessel. The graphs capture snapshots of the variation in the vessel diameter with position, x, along the vessel. However, when visualized over time, the variations in the vessel diameter with position, x, along the vessel, propagate as pulses along the vessel. For instance, as may be seen best in Fig. 4, a peak of the vessel deformation pulse 150 is located at the position $x_0$ along the vessel at time $T_0$. Over time, the peak of the vessel deformation pulse 150 propagates along the vessel. Thus, the peak of the vessel deformation pulse 150 is located at the position $x_1$, and $x_2$, at the later times $T_1$, and $T_2$ respectively. The vessel deformation pulse propagates along the vessel with a so-called "pulse wave velocity". The vessel deformation pulse has an instantaneous velocity $v(x_0, T_0)$ at the position $x_0$ and at the time $T_0$, an instantaneous velocity $v(x_1, T_1)$ at the position $x_1$ and at the time $T_1$, and an instantaneous velocity $v(x_2, T_2)$ at the position $x_2$ and at the time $T_2$. The vessel deformation pulse may also be considered to have an average velocity $v_{average}(x_0 \rightarrow x_2, T_0 \rightarrow T_2)$ between the positions $x_0$ and $x_2$, over the corresponding time interval between $T_0$ and $T_2$.

The vessel pulse wave velocity "PWV" facilitates an evaluation of the stiffness of the vessel via the Moens-Korteweg equation:

$$PWV = \sqrt{\frac{E_{inc} \cdot h}{2 \cdot r \cdot \rho}} \qquad \qquad \text{Equation 1}$$

wherein $E_{inc}$ is the incremental elastic modulus of the vessel wall, $h$ is the vessel wall thickness, and $r$ is the vessel radius, and $\rho$ is the density of blood. Thus, the value of the pulse wave velocity, PWV, as determined by analyzing the temporal sequence of images in the operation S120, i.e. based on the temporal variation in a cross sectional measurement, such as the diameter, of the vessel along a portion of the vessel and over the at least a portion of a cardiac cycle, may be used to evaluate the stiffness of the vessel, i.e. the parameter $E_{inc}$, the incremental elastic modulus of the vessel wall, using Equation 1.

The velocity of the vessel deformation pulse may be determined by analyzing the temporal sequence of images using known image processing techniques. For instance, the peak of the vessel deformation pulse may be tracked between any pair of images in the temporal sequence in order to determine a distance travelled by the pulse between the images, and the distance may be used in combination with the imaging frame rate to calculate a velocity of the vessel deformation pulse between the images.

As described above with reference to Fig. 4, the vessel deformation pulse has an instantaneous velocity at any given position along the vessel. The instantaneous velocity may be used to evaluate, via Equation 1, a corresponding local value for the $E_{inc}$, i.e. the stiffness, at any desired position along the vessel. This provides detailed information on the stiffness of the vessel along its length that may facilitate an improved assessment of a vascular condition. Thus, in one example, the operation of evaluating at least one mechanical property 140 of the vessel 110, comprises i) evaluating the at least one mechanical property 140 of the vessel 110 at a plurality of the positions $x_0$, $x_2$, along the portion of the vessel based on the velocity $v(x_0, T_0)$, $v(x_2, T_2)$ of the vessel deformation pulse 150 at the corresponding positions. In this example, the outputting operation S130 comprises i) outputting the value of the at least one mechanical property 140 of the vessel 110 at the plurality of the positions $x_0$, $x_2$, along the portion of the vessel.

As also described above with reference to Fig. 4, the vessel deformation pulse may also be considered to have an average velocity between any two positions along the vessel. Thus, in another example, the operation of evaluating at least one mechanical property 140 of the vessel 110, comprises ii) evaluating an average value of the at least one mechanical property 140 of the vessel 110 along the portion $x_0 \rightarrow x_2$ of the vessel based on the velocity $v_{average}(x_0 \rightarrow x_2, T_0 \rightarrow T_2)$ of the vessel deformation pulse 150 between the positions defining the portion of the vessel. In this example, the outputting operation S130 comprises ii) outputting the average value of the at least one mechanical property 140 of the vessel 110.

Instead of evaluating the mechanical property(ies) of the vessel in the operation S120 using Equation 1, other techniques may alternatively be used. For instance, in another example, a mechanical property(ies) of a vessel may be evaluated by inputting the X-ray data representing the temporal sequence of images capturing the vessel 110 over at least a portion of a cardiac cycle into a trained machine learning algorithm. In response to the inputting, the trained machine learning algorithm may output the mechanical property(ies) of the vessel. In this example, the training data that is used to train the machine learning algorithm may be provided by mechanical properties that are derived from pulse wave velocity measurements of vessels that are calculated using Equation 1 above from Doppler ultrasound imaging data instead of X-ray data.

As mentioned above, the images in the temporal sequence may be mutually registered in order to improve the accuracy of the cross-sectional measurements of the vessel that are analyzed in the operation S120. Thus, in one example, the operation of analyzing S120 the X-ray data 130 comprises mutually registering the images in the temporal sequence. In this example, the at least one mechanical property 140 of the vessel 110 is evaluated based on the temporal variation in the cross sectional measurement of the vessel along the portion of the vessel in the mutually-registered images over the at least a portion of a cardiac cycle.

In this example, the mutual registration may be performed by registering the images in the temporal sequence to a selected image in the temporal sequence, or by registering the images in the temporal sequence to another reference image that represents the vessel, such as a volumetric, or projection, angiographic image representing the vessel. The images may be mutually registered based on a reference in the vessel in the images, such as landmark (e.g. a vessel bifurcation, or a calcium deposit), the vessel centerline, and so forth. Various registration techniques may be used to perform this registration. The registration may include a non-rigid and/or a rigid registration. An example of a registration technique that may be used perform this registration in a cardiac vessel undergoing cardiac motion is described in the document WO2012/107857A1.

In one example, the operation of analyzing S120 the X-ray data 130 includes applying a foreshortening correction to the vessel 110 to correct a velocity of the vessel deformation pulse 150 propagating along the vessel in an out-of-plane direction with respect to a plane of the images in the temporal sequence.

Foreshortening refers to the deformation of an object in an X-ray projection image that is caused by the object not being arranged parallel to a plane of an X-ray detector. In the context of an X-ray projection image of a vessel, foreshortening arises when the path of the vessel deviates from a plane that is parallel to the plane of the X-ray detector. Foreshortening results in inaccuracies in vessel dimensions in X-ray projection images (e.g. inaccuracies in the vessel length, or the vessel diameter) which if uncorrected can lead to inaccuracies in the velocity of the vessel deformation pulse that was described with reference to Fig. 3. This, in-turn can lead to an inaccuracy in the mechanical property(ies) that are calculated from such velocities.

In this example, a more accurate value of the mechanical property(ies) of the vessel is provided by applying the foreshortening correction to the vessel. The foreshortening correction may be applied in various ways. For instance, in one example, a foreshortening correction may be calculated for the vessel based on an assumed shape of the vessel and a known geometry of the X-ray imaging system. The orientation of the vessel with respect to the geometry of the X-ray imaging system is used to calculate a scaling factor that varies along the vessel path in accordance with deviations of the vessel path from a plane that is parallel to the plane of the X-ray detector. The foreshortening correction may be applied to the images in the temporal sequence in order to provide a local correction to the dimensions of the vessel.

In another example, a foreshortening correction may be applied to the vessel by registering the images in the temporal sequence to a volumetric angiographic image of the vessel, or to a plurality of projection angiographic images of the vessel.

In this example, the one or more processors 120 are configured to:

receive angiographic data comprising a volumetric angiographic image of the vessel 110, or a plurality of projection angiographic images of the vessel 110, the plurality of projection angiographic images representing the vessel from different viewing angles with respect to the vessel;

register the images in the temporal sequence to the volumetric angiographic image, or to the plurality of projection angiographic images, respectively; and

wherein the analyzing S120 the X-ray data 130 comprises evaluating the at least one mechanical property 140 of the vessel 110 based on the temporal variation in the cross sectional measurement of the vessel along the portion of the vessel in the registered images.

In this example, volumetric image of the vessel, and likewise the projection angiographic images representing the vessel from different viewing angles with respect to the vessel, include depth information relating to the vessel. Registering the images in the temporal sequence to the volumetric angiographic image, or to the plurality of projection angiographic images, has the effect of deforming the vessel such that the vessel dimensions, e.g. the vessel length and diameter, are accurately represented in the registered images. The images may be mutually registered based on a reference in the vessel in the images, such as landmark (e.g. a vessel bifurcation, or a calcium deposit), the vessel centerline, and so forth. Various registration techniques may be used to perform this registration. The registration may include a non-rigid and/or a rigid registration. An example of a registration technique that may be used perform this registration in a cardiac vessel undergoing cardiac motion is described in the document WO2012/107857A1.

In this example, the registering operation may include deforming a shape of the vessel 110 in the images in the temporal sequence in a direction perpendicular to a plane of the images in the temporal sequence such that the foreshortening correction is applied by performing the registering.

Thus, the registering operation in this example has the effect of applying a foreshortening correction to the vessel 110. Since the operation of analyzing S120 the X-ray data 130 comprises evaluating the at least one mechanical property 140 of the vessel 110 based on the temporal variation in the cross sectional measurement of the vessel along the portion of the vessel in the registered images, this has the effect of correcting the velocity of the vessel deformation pulse 150 propagating along the vessel in an out-of-plane direction with respect to a plane of the images in the temporal sequence. Thus, it results in a more accurate evaluation of the mechanical property(ies) 140 of the vessel 110.

In another example, a viewing angle for viewing the vessel is determined. In this example, the one or more processors 120 are configured to:

analyze the volumetric angiographic image of the vessel 110, or the plurality of projection angiographic images of the vessel, respectively, to determine a viewing angle for viewing the vessel; and

output an indication of the viewing angle.

The viewing angle may be determined based on various factors. For example, it may be based on the provision of a projected image of the vessel with minimal foreshortening and/or a the provision of a projected image of the vessel having a minimal number of vessels that overlap with the vessel. The viewing angle may be determined for a selected vessel represented in the volumetric angiographic image of the vessel, or for a selected vessel in the plurality of projection angiographic images of the vessel, by evaluating a vessel overlap metric and/or a vessel foreshortening metric for the vessel in a virtual projection image of the vessel that is generated by projecting the vessel in the respective angiographic image(s) onto a virtual X-ray detector using virtual X-rays emitted by a virtual X-ray source. The viewing angle may then be determined by optimizing the value of the foreshortening metric(s) based on the viewing angle.

In another example, the one or more processors 120 are configured to extract one or more geometric parameters of the vessel 110 from the images in the temporal sequence. The one or more geometric parameters include a thickness measurement of the vessel in a direction parallel to and/or perpendicular to a plane of the images in the temporal sequence. The thickness measurement of the vessel in the direction perpendicular to the plane of the images in the temporal sequence is determined based on an X-ray attenuation of the vessel in the images in the temporal sequence.

This example provides, from projection images, a thickness measurement of the vessel in the direction perpendicular to the plane of the images in the temporal sequence. The thickness measurement may be used for various purposes, including to determine, based on its temporal variation along the portion of the vessel and over the at least a portion of a cardiac cycle, the value of the at least one mechanical property 140 of the vessel 110. The thickness measurement may alternatively be used improve the registration of the images in the temporal sequence to an angiographic image, and or to simulate a blood flow in the vessel, or to evaluate a haemodynamic parameter for the vessel, as described in more detail below.

In another example, the mechanical property(ies) of the vessel that are evaluated based on the temporal variation in the cross sectional measurement of the vessel along the portion of the vessel in the registered images, are illustrated in a graphical representation. In this example, the one or more processors 120 are configured to output a graphical representation of the vessel 110 including an indication of a correspondence between the value of the at least one mechanical property 140 and a position of the value in the vessel in the graphical representation. The graphical representation is generated based on the volumetric angiographic image of the vessel, or the plurality of projection angiographic images of the vessel, respectively; and the correspondence is determined based on the registering.

In this example, the graphical representation may be provided in the form of an overlay image wherein the values of the one mechanical property(ies) of the vessel are overlaid onto the a graphical representation of the vessel that is generated from the angiographic images, for example. By providing the mechanical property(ies) of the vessel in this manner, a physician may readily perform a diagnosis of the vessel and/or select a treatment option for the vessel. For instance, the graphical representation may illustrate portions of the vessel having relatively higher stiffness values. The physician may then use the graphical representation to assess the suitability of a renal sympathetic denervation treatment procedure for the vessel based on the stiffness values, as described above. The graphical representation may also be used as input to a model that is used to simulate a blood flow in the vessel and/or evaluate a haemodynamic parameter for the vessel, as described in more detail below.

In another example, the mechanical property(ies) of the vessel are illustrated in a graphical representation. In contrast to the example described above in which it is the mechanical property(ies) of the vessel that are evaluated based on the temporal variation in the cross sectional measurement of the vessel along the portion of the vessel in the registered images that are illustrated in a graphical representation, in this example it is the mechanical property(ies) of the vessel that are evaluated based on the temporal variation in the cross sectional measurement of the vessel along the portion of the vessel in the images that are illustrated in a graphical representation. In other words, in this example, the mechanical property(ies) of the vessel are not evaluated from images that are registered to an angiographic image. A graphical representation is however generated using an angiographic image. In this example, the one or more processors 120 are configured to:

receive angiographic data comprising a volumetric angiographic image of the vessel 110, or a plurality of projection angiographic images of the vessel, the plurality of projection angiographic images representing the vessel from different viewing angles with respect to the vessel;

register the images in the temporal sequence to the volumetric angiographic image or to the plurality of projection angiographic images, respectively, to determine a correspondence between a position of the value of the at least one mechanical property 140 in the images in the temporal sequence and a position of the value of the at least one mechanical property in the volumetric angiographic image, or in the plurality of projection angiographic images, respectively; and

output a graphical representation of the vessel 110 including an indication of the correspondence;
wherein the graphical representation is generated based on the volumetric angiographic image, or the plurality of projection angiographic images, respectively; and
wherein the correspondence is determined based on the registering.

This example has similar advantages to the alternative example described above. For instance, by providing the mechanical property(ies) of the vessel in this manner, a physician may readily perform a diagnosis of the vessel and/or select a treatment option for the vessel.

In another example, a blood flow velocity in the vessel is calculated from the temporal sequence of images. The blood flow velocity provides additional information that may facilitate a physician to assess a vascular condition and/or select a treatment option for the vessel. In this example, the temporal sequence of images is generated subsequent to a contrast agent injection into the vessel 110, and the one or more processors 120 are configured to:

analyze the images in the temporal sequence to determine a temporal velocity profile $VCA_2(t)$ of the contrast agent along the vessel 110 based on temporal variations in an intensity gradient $IG(t)$ of the contrast agent along the portion of the vessel in the images in the temporal sequence;
evaluate a blood flow velocity in the vessel 110 based on the temporal velocity profile $VCA_2(t)$; and
output the value of the blood flow velocity.

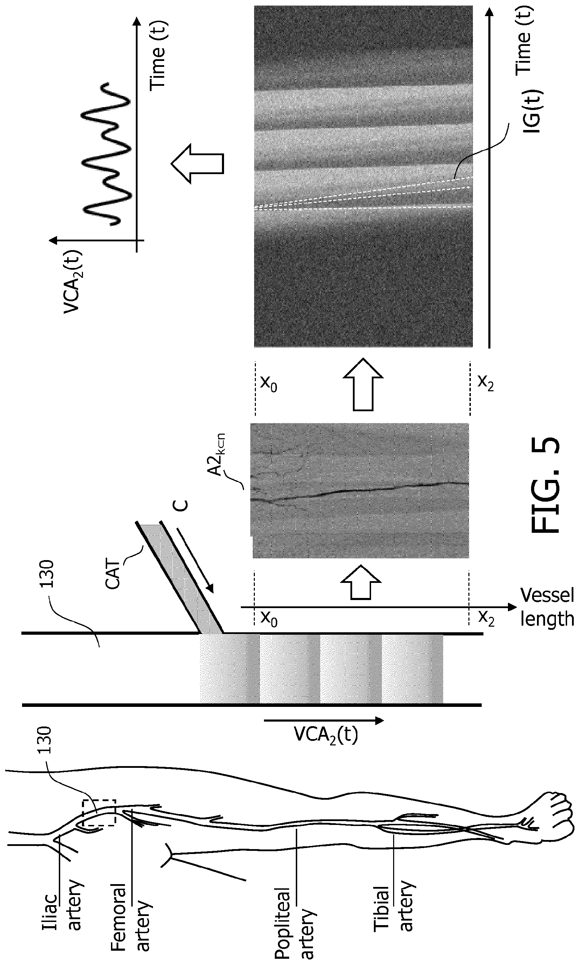

This example is described with reference to Fig. 5, which is a schematic diagram illustrating an example of a technique for determining a temporal velocity profile $VCA_2(t)$ of a contrast agent along a vessel 130, in accordance with some aspects of the present disclosure. The technique used in this example is described in more detail for a static vessel in the document by Pereira V. M., et al. entitled "A DSA-based Method Using Contrast-Motion Estimation for the Assessment of the Intra-Aneurysmal Flow Changes Induced by Flow-Diverter Stents", American Journal of Neuroradiology, 2013, 34(4), pages 808-815. In Fig. 5, the example is given for determining a temporal velocity profile $VCA_2(t)$ in the femoral artery. However, the technique may also be used to determine the temporal velocity profile $VCA_2(t)$ in other vessels, including in cardiac vessels, and in which vessel motion presents additional challenges.

With reference to Fig. 5, starting on the left-hand side of the Figure, a contrast agent, is injected into the vessel 130 by means of a catheter CAT. The contrast agent has a contrast agent injection rate labelled "C" which remains constant during the injection. The contrast agent injection rate that is used in the contrast agent injection may be a fraction of the average expected hyperemic flow rate in the vessel. Consequently, the pulsatile flow of the blood, dilutes the contrast agent in accordance with the blood's temporal velocity. This results in a contrast agent that at any instant in time has an intensity at positions along the vessel that varies in accordance with the blood's temporal velocity at the time of the injection. In Fig. 5, an angiographic image $A2_{k \subset n}$ from the temporal sequence of images $A2_{1..n}$ captures a profile of the contrast agent distribution along the lumen of the vessel 130. The profile is indicated in Fig. 5 for positions between xo and xz in the image $A2_{k \subset n}$. The profile is then computed along the centerline of the image $A2_{k \subset n}$, the centerline having been provided by the registration operation described above with reference to Fig. 3. In the lower right-hand portion of Fig. 5, the profile along the centerline is plotted for each of multiple angiographic images $A2_{1..n}$, each image corresponding to a vertical line in the illustration in the lower right-hand portion of Fig. 5. An intensity gradient in this Figure, $IG(t)$ may then be computed, and this gradient represents the velocity of a front of the contrast agent as it flows along the lumen. This gradient $IG(t)$, i.e. the velocity of the front, can then be plotted as a function of time, resulting in the temporal velocity profile $VCA_2(t)$ of the contrast agent illustrated in the upper right-hand portion of Fig. 5.

Determining the temporal velocity profile $VCA_2(t)$ in this manner allows for the provision of a temporal velocity profile $VCA_2(t)$ from angiographic images, i.e. in a non-invasive manner.

As mentioned above, the mechanical property(ies) of the vessel that are calculated by the system 100 described above may be used for various purposes. In one example, the one or more processors 120 are configured to:

simulate, based on the value of the at least one mechanical property 140 of the vessel 110, a blood flow in the vessel; and/or
evaluate a haemodynamic parameter for the vessel 110; and
output an indication of the simulated blood flow and/or the value of the haemodynamic parameter, respectively.

The blood flow, and likewise the haemodynamic parameter(s) that are evaluated in this example may be used to assess vascular conditions. Their values may be determined using a hemodynamic model that incorporates the value(s) of the mechanical property(ies) of the vessel. The haemodynamic model may be solved using computational Fluid Dynamics "CFD" techniques. For instance, a haemodynamic model may be generated that represents the geometry of the vessel 110 via its 3D centerline with elliptic cross-sections along a length of the 3D centerline. The haemodynamic model may incorporate the value(s) of the mechanical property(ies) of the vessel as a distribution of values along the 3D centerline.

The hemodynamic model may be based on Navier-Stokes partial differential equations. The model may then be solved using CFD techniques to simulate the blood flow in the vessel and to evaluate various haemodynamic parameter(s).

In this example, the blood flow and the haemodynamic parameter(s) values are evaluated using vessel-specific values for the mechanical property(ies) of the vessel. Conventionally, the blood flow, and likewise the haemodynamic parameter(s), are simulated using population-averaged values for the mechanical property(ies) of a vessel. This limits their accuracy. By contrast, since in this example, the blood flow, and likewise the haemodynamic parameter(s), are evaluated using vessel-specific values for the mechanical property(ies), the system provides the blood flow, and likewise the haemodynamic parameter(s), with improved accuracy. This, in-turn, facilitates a physician to make a more reliable assessment of a vascular condition and to make an improved selection of their treatment options.

In this example, the haemodynamic model may be used to evaluate haemodynamic parameters such as the Fractional Flow Reserve " FFR". The FFR is defined as the ratio of the distal pressure to the proximal pressure within a vessel. The pressure values that are used to calculate the FFR value may be determined from the haemodynamic model mentioned above, as described in the document US8157742. Other examples of haemodynamic parameters that may be evaluated in this example include the Coronary Flow Reserve "CFR", the Thrombolysis in Myocardial Infarction "TIMI" flow grade, TIMI frame count, and the Index of Microvascular Resistance "IMR" or the Hyperemic Microvascular Resistance index "HMR".

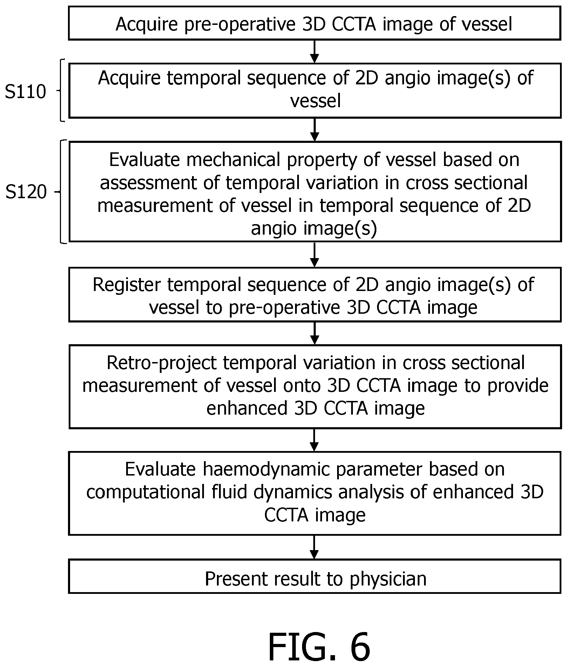

An example illustrating the evaluation of a haemodynamic parameter is shown in Fig. 6, which is a flowchart illustrating an example of a method of evaluating a haemodynamic parameter for a vessel based on a value of a mechanical property of the vessel, in accordance with some aspects of the present disclosure. The example illustrated in Fig. 6 describes the various steps outlined above, and results in the presentation of the result to a physician.

In a related example, the one or more processors 120 are configured to extract one or more geometric parameters of the vessel 110 from the volumetric angiographic image, or from the plurality of angiographic images, respectively. In this example, the one or more processors 120 are configured to simulate the blood flow in the vessel and/or evaluate the haemodynamic parameter for the vessel, based further on the one or more geometric parameters.

In this example, since the geometric parameter(s) are extracted from the angiographic image(s), the need to perform invasive techniques for obtaining these parameters is obviated. The one or more geometric parameters may include a thickness measurement of the vessel in a direction parallel to and/or perpendicular to a plane of the images in the temporal sequence. The thickness measurement of the vessel in the direction perpendicular to the plane of the images in the temporal sequence may be determined based on an X-ray attenuation of the vessel in the images in the temporal sequence. This was described above. Thus, the one or more processors 120 may simulate the blood flow in the vessel 110 and/or evaluate the haemodynamic parameter for the vessel, based further on the thickness measurement(s) of the vessel.

In another example, the one or more processors 120 are configured to receive one or more of:

the value of the blood flow velocity described above;
pressure data representing a pressure in the vessel 110; and
wherein the one or more processors 120 are configured to simulate the blood flow in the vessel 110 and/or evaluate the haemodynamic parameter for the vessel 110, based further on the value of the blood flow velocity and/or the pressure, respectively.

In this example, the blood flow velocity and the pressure data are used as boundary conditions in the haemodynamic model that is used to simulate the blood flow in the vessel 110 and/or evaluate the haemodynamic parameter. The value of the blood flow velocity may be determined as described above with reference to Fig. 5; i.e. by analyzing the images in the temporal sequence to determine a temporal velocity profile $VCA_2(t)$ of the contrast agent along the vessel 110. Using the blood flow velocity in this manner improves the accuracy of the haemodynamic model without the need to obtain its measurement using invasive techniques. The pressure data that is received in this example may be obtained from a contrast agent injector that delivers the contrast agent in technique described above with reference to Fig. 5. Using the pressure data in this manner likewise improves the accuracy of the haemodynamic model.

In another example, the vessel 110 is a cardiac vessel, and the one or more processors 120 are configured to evaluate a myocardial bridging metric for the vessel. The myocardial bridging metric represents a likelihood of the vessel being subjected to myocardial bridging. The myocardial bridging metric is evaluated based on the simulated blood flow and/or the value of the haemodynamic parameter. The one or more processors 120 are configured to output an indication of the value of the myocardial bridging metric.

The myocardial bridging metric may be calculated based on the relaxation properties of the vessel during the diastole phase, e.g. by evaluating the stiffness of the vessel during the diastole phase, for example. The myocardial bridging metric may be outputted in various ways. For instance, the value of the myocardial bridging metric may be outputted as an overlay onto an angiographic image representing the vessel. By evaluating, and outputting, the myocardial bridging metric, the system facilitates a physician to assess the severity of this vascular condition, and also to select a treatment option.

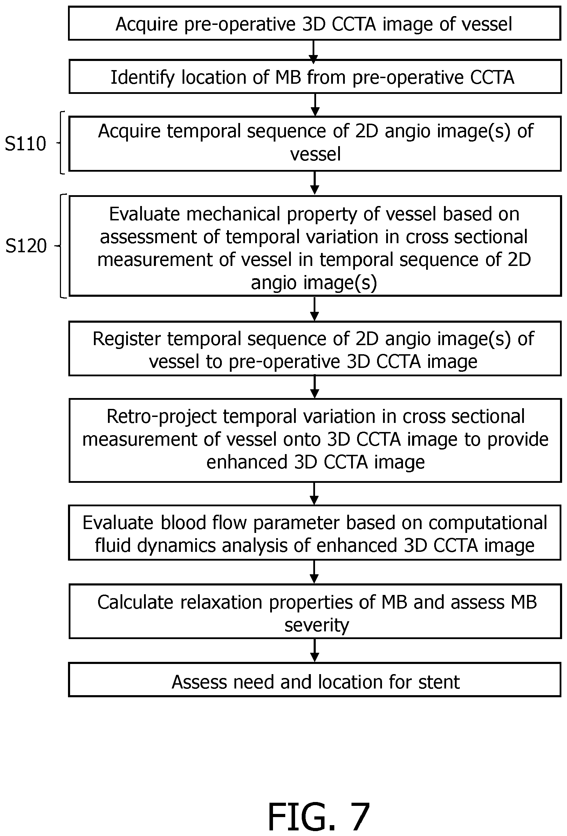

An example of the evaluation of a myocardial bridging metric is illustrated in Fig. 7, which is a flowchart illustrating an example of a method of evaluating a myocardial bridging metric for a vessel based on a value of a mechanical property of the vessel, in accordance with some aspects of the present disclosure. The example illustrated in Fig. 7 describes the various steps above, and results in an assessment of the severity of the myocardial bridge. A physician, or the system, may then assess the need and location for a stent for treating the myocardial bridge.

In another example, the temporal sequence of images captures the vessel 110 over at least a portion of the diastole phase of the cardiac cycle such that the analyzing S120 comprises evaluating the at least one mechanical property 140 of the vessel 110 during a relaxation phase of the vessel. In this example, the value of the myocardial bridging metric is evaluated during the relaxation phase of the vessel.

As mentioned above, the stiffness of the vessel during the relaxation phase of the vessel may be used to evaluate the myocardial bridging metric.

In another example, the one or more processors 120 are configured to evaluate, based on the calculated blood flow in the vessel 110, a suitability of one or more treatment procedures for treating the vessel; and output an indication of the suitability of the one or more treatment procedures.

In this example, the one or more processors 120 may evaluate the suitability of a stenting procedure for treating a cardiac vessel that has a myocardial bridge, for example. For instance, if the calculated blood flow in the vessel 110 is below a threshold value, treatment may be deemed suitable. The location of a stent for treating the myocardial bridge may be identified automatically in the vessel based on the extent of the vessel for which the stiffness of the vessel satisfies a threshold condition.

In another example, the one or more processors 120 are configured to evaluate a suitability of a stenting procedure for the vessel 110 and/or evaluate a recommended position for a stent in the vessel 110; and to output an indication of the suitability of a stenting procedure and/or the recommended position for the stent in the vessel 110, respectively.

In this example, the one or more processors 120 may evaluate the suitability of the stenting procedure if the calculated blood flow in the vessel 110 satisfies a threshold condition. The location of the stent may be identified automatically in the vessel based on the vessel lumen diameter. For instance, the location of the stent may be identified such that it covers a portion of the vessel along which a diameter of the vessel lumen is below a threshold value.

It is noted that in addition to the one or more processors 120, the system 100 may also include one or more of: an X-ray imaging system for providing the X-ray data 130, such as for example the projection X-ray imaging system 170 illustrated in Fig. 1; a display, such as the monitor 160 illustrated in Fig. 1, for displaying the value of the mechanical property(ies) 140 of the vessel 110, the temporal sequence of images of the vessel, other outputs generated by the one or more processors 120, and so forth; a contrast agent injector (not illustrated in Fig. 1) for injecting a contrast agent into the vessel; a patient bed 180; and a user input device (not illustrated in Fig. 1) configured to receive user input in relation to the operations performed by the one or more processors 120, such as a keyboard, a mouse, a touchscreen, and so forth.

In another example, a computer-implemented method of evaluating a property of a vessel 110, is provided. The method comprises:

receiving S110 X-ray data 130 representing a temporal sequence of images of the vessel 110, the temporal sequence of images capturing the vessel 110 over at least a portion of a cardiac cycle;
analyzing S120 the X-ray data 130 to evaluate at least one mechanical property 140 of the vessel 110, the at least one mechanical property of the vessel being evaluated based on a temporal variation in a cross sectional measurement B(x, t) of the vessel along a portion of the vessel and over the at least a portion of a cardiac cycle; and
outputting S130 the value of the at least one mechanical property 140 of the vessel 110.

In another example, a computer program product, is provided. The computer program product comprises instructions which when executed by one or more processors, cause the one or more processors to carry out a method of evaluating a property of a vessel 110. The method comprises:

receiving S110 X-ray data 130 representing a temporal sequence of images of the vessel 110, the temporal sequence of images capturing the vessel 110 over at least a portion of a cardiac cycle;
analyzing S120 the X-ray data 130 to evaluate at least one mechanical property 140 of the vessel 110, the at least one mechanical property of the vessel being evaluated based on a temporal variation in a cross sectional measurement B(x, t) of the vessel along a portion of the vessel and over the at least a portion of a cardiac cycle; and
outputting S130 the value of the at least one mechanical property 140 of the vessel 110.

The above examples are to be understood as illustrative of the present disclosure, and not restrictive. Further examples are also contemplated. For instance, the examples described in relation to the system 100, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system (100) for evaluating a property of a vessel (110), the system comprising one or more processors (120) configured to:

   receive (S110) X-ray data (130) representing a temporal sequence of images of the vessel (110), the temporal sequence of images capturing the vessel (110) over at least a portion of a cardiac cycle;
   analyze (S120) the X-ray data (130) to evaluate at least one mechanical property (140) of the vessel (110), the at least one mechanical property of the vessel being evaluated based on a temporal variation in a cross sectional measurement ($B(x, t)$) of the vessel along a portion of the vessel and over the at least a portion of a cardiac cycle; and
   output (S130) the value of the at least one mechanical property (140) of the vessel (110).

2. The system according to claim 1, wherein the analyzing (S120) the X-ray data (130) comprises determining, based on the temporal variation in the cross sectional measurement ($B(x, t)$) of the vessel at each of a plurality of positions ($x_0$, $x_2$) along the portion of the vessel and over the at least a portion of a cardiac cycle, a velocity ($v(x_0, T_0)$, $v(x_2, T_2)$, $v_{average}(x_0{\rightarrow}x_2, T_0{\rightarrow}T_2)$) of a vessel deformation pulse (150) propagating along the vessel (110); and wherein the evaluating is performed based on the velocity ($v(x_0, T_0)$, $v(x_2, T_2)$, $v_{average}(x_0{\rightarrow}x_2, T_0{\rightarrow}T_2)$) of the vessel deformation pulse (150).

3. The system according to claim 2, wherein the evaluating comprises i) evaluating the at least one mechanical property (140) of the vessel (110) at a plurality of the positions ($x_0$, $x_2$) along the portion of the vessel based on the velocity ($v(x_0, T_0)$, $v(x_2, T_2)$) of the vessel deformation pulse (150) at the corresponding positions, or ii) evaluating an average value of the at least one mechanical property (140) of the vessel (110) along the portion ($x_0{\rightarrow}x_2$) of the vessel based on the velocity ($v_{average}(x_0{\rightarrow}x_2, T_0{\rightarrow}T_2)$) of the vessel deformation pulse (150) between the positions defining the portion of the vessel; and wherein the outputting (S130) comprises i) outputting the value of the at least one mechanical property (140) of the vessel (110) at the plurality of the positions ($x_0$, $x_2$) along the portion of the vessel, or ii) outputting the average value of the at least one mechanical property (140) of the vessel (110), respectively.

4. The system according to any previous claim, wherein the X-ray data (130) represents a temporal sequence of projection images of the vessel (110).

5. The system according to any previous claim, wherein the X-ray data (130) represents a temporal sequence of projection images of the vessel (110) from each of a plurality of different viewing angles with respect to the vessel.

6. The system according to claim 2, wherein the analyzing (S120) the X-ray data (130) comprises applying a foreshortening correction to the vessel (110) to correct a velocity of the vessel deformation pulse (150) propagating along the vessel in an out-of-plane direction with respect to a plane of the images in the temporal sequence.

7. The system according to any previous claim, wherein the one or more processors (120) are further configured to:

   receive angiographic data comprising a volumetric angiographic image of the vessel (110), or a plurality of projection angiographic images of the vessel (110), the plurality of projection angiographic images representing the vessel from different viewing angles with respect to the vessel;
   register the images in the temporal sequence to the volumetric angiographic image, or to the plurality of projection angiographic images, respectively; and

wherein the analyzing (S120) the X-ray data (130) comprises evaluating the at least one mechanical property (140) of the vessel (110) based on the temporal variation in the cross sectional measurement of the vessel along the portion of the vessel in the registered images.

8. The system according to claim 7, wherein the one or more processors (120) are further configured to output a graphical representation of the vessel (110) including an indication of a correspondence between the value of the at least one mechanical property (140) and a position of the value in the vessel in the graphical representation;

wherein the graphical representation is generated based on the volumetric angiographic image of the vessel, or the plurality of projection angiographic images of the vessel, respectively; and
wherein the correspondence is determined based on the registering.

9. The system according to any one of claims 1-6, wherein the one or more processors (120) are further configured to:

receive angiographic data comprising a volumetric angiographic image of the vessel (110), or a plurality of projection angiographic images of the vessel, the plurality of projection angiographic images representing the vessel from different viewing angles with respect to the vessel;
register the images in the temporal sequence to the volumetric angiographic image or to the plurality of projection angiographic images, respectively, to determine a correspondence between a position of the value of the at least one mechanical property (140) in the images in the temporal sequence and a position of the value of the at least one mechanical property in the volumetric angiographic image, or in the plurality of projection angiographic images, respectively; and
output a graphical representation of the vessel (110) including an indication of the correspondence;
wherein the graphical representation is generated based on the volumetric angiographic image, or the plurality of projection angiographic images, respectively; and
wherein the correspondence is determined based on the registering.

10. The system according to any previous claim, wherein the temporal sequence of images is generated subsequent to a contrast agent injection into the vessel (110), and wherein the one or more processors (120) are further configured to:

analyze the images in the temporal sequence to determine a temporal velocity profile ($VCA_2(t)$) of the contrast agent along the vessel (110) based on temporal variations in an intensity gradient ($IG(t)$) of the contrast agent along the portion of the vessel in the images in the temporal sequence;
evaluate a blood flow velocity in the vessel (110) based on the temporal velocity profile ($VCA_2(t)$); and
output the value of the blood flow velocity.

11. The system according to any previous claim, wherein the one or more processors (120) are further configured to:

simulate, based on the value of the at least one mechanical property (140) of the vessel (110), a blood flow in the vessel;
and/or
evaluate a haemodynamic parameter for the vessel (110); and
output an indication of the simulated blood flow and/or the value of the haemodynamic parameter, respectively.

12. The system according to claim 11 when dependent on any one of claims 7-9, wherein the one or more processors (120) are further configured to extract one or more geometric parameters of the vessel (110) from the volumetric angiographic image, or from the plurality of angiographic images, respectively; and
wherein the one or more processors (120) are configured to simulate the blood flow in the vessel and/or evaluate the haemodynamic parameter for the vessel, based further on the one or more geometric parameters.

13. The system according to claim 11, wherein the one or more processors (120) are further configured to receive one or more of:

the value of the blood flow velocity according to claim 10;
pressure data representing a pressure in the vessel (110); and
wherein the one or more processors (120) are configured to simulate the blood flow in the vessel (110) and/or evaluate the haemodynamic parameter for the vessel (110), based further on the value of the blood flow velocity and/or the pressure, respectively.

**14.** The system according to any one of claims 11 - 13, wherein the vessel (110) is a cardiac vessel, and wherein the one or more processors (120) are further configured to evaluate a myocardial bridging metric for the vessel, the myocardial bridging metric representing a likelihood of the vessel being subjected to myocardial bridging, and wherein the myocardial bridging metric is evaluated based on the simulated blood flow and/or the value of the haemodynamic parameter; and

wherein the one or more processors (120) are further configured to output an indication of the value of the myocardial bridging metric.

**15.** A computer-implemented method of evaluating a property of a vessel (110), the method comprising:

receiving (S110) X-ray data (130) representing a temporal sequence of images of the vessel (110), the temporal sequence of images capturing the vessel (110) over at least a portion of a cardiac cycle;
analyzing (S120) the X-ray data (130) to evaluate at least one mechanical property (140) of the vessel (110), the at least one mechanical property of the vessel being evaluated based on a temporal variation in a cross sectional measurement (B(x, t)) of the vessel along a portion of the vessel and over the at least a portion of a cardiac cycle; and
outputting (S130) the value of the at least one mechanical property (140) of the vessel (110).

## FIG. 1

## FIG. 2

FIG. 3

FIG. 4

17

FIG. 5

| Acquire pre-operative 3D CCTA image of vessel |

S110

| Acquire temporal sequence of 2D angio image(s) of vessel |

S120

| Evaluate mechanical property of vessel based on assessment of temporal variation in cross sectional measurement of vessel in temporal sequence of 2D angio image(s) |

| Register temporal sequence of 2D angio image(s) of vessel to pre-operative 3D CCTA image |

| Retro-project temporal variation in cross sectional measurement of vessel onto 3D CCTA image to provide enhanced 3D CCTA image |

| Evaluate haemodynamic parameter based on computational fluid dynamics analysis of enhanced 3D CCTA image |

| Present result to physician |

# FIG. 6

```
┌──────────────────────────────────────────────────────┐
│          Acquire pre-operative 3D CCTA image of vessel          │
└──────────────────────────────────────────────────────┘
                              ↓
┌──────────────────────────────────────────────────────┐
│          Identify location of MB from pre-operative CCTA         │
└──────────────────────────────────────────────────────┘
                              ↓
```

S110 — ┌──────────────────────────────────────────────────────┐
       │       Acquire temporal sequence of 2D angio image(s) of       │
       │                            vessel                             │
       └──────────────────────────────────────────────────────┘
                              ↓

S120 — ┌──────────────────────────────────────────────────────┐
       │          Evaluate mechanical property of vessel based on       │
       │        assessment of temporal variation in cross sectional      │
       │        measurement of vessel in temporal sequence of 2D         │
       │                        angio image(s)                         │
       └──────────────────────────────────────────────────────┘
                              ↓

```
┌──────────────────────────────────────────────────────┐
│       Register temporal sequence of 2D angio image(s) of       │
│              vessel to pre-operative 3D CCTA image            │
└──────────────────────────────────────────────────────┘
                              ↓
┌──────────────────────────────────────────────────────┐
│        Retro-project temporal variation in cross sectional      │
│       measurement of vessel onto 3D CCTA image to provide      │
│                    enhanced 3D CCTA image                     │
└──────────────────────────────────────────────────────┘
                              ↓
┌──────────────────────────────────────────────────────┐
│       Evaluate blood flow parameter based on computational      │
│        fluid dynamics analysis of enhanced 3D CCTA image       │
└──────────────────────────────────────────────────────┘
                              ↓
┌──────────────────────────────────────────────────────┐
│        Calculate relaxation properties of MB and assess MB      │
│                           severity                           │
└──────────────────────────────────────────────────────┘
                              ↓
┌──────────────────────────────────────────────────────┐
│             Assess need and location for stent               │
└──────────────────────────────────────────────────────┘
```

# FIG. 7

**EP 4 681 646 A1**

# EUROPEAN SEARCH REPORT

**Application Number**

EP 24 30 6190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2017/132788 A1 (VENUGOPAL PREM [US] ET AL) 11 May 2017 (2017-05-11)<br>* abstract *<br>* paragraph [0001] - paragraph [0006] *<br>* paragraph [0033] - paragraph [0039] *<br>* paragraph [0043] - paragraph [0045] *<br>* figure 9 *<br>----- | 1-15 | INV.<br>A61B6/00 |
| A | EP 3 832 662 A1 (KONINKLIJKE PHILIPS NV [NL]) 9 June 2021 (2021-06-09)<br>* abstract *<br>* paragraph [0001] - paragraph [0003] *<br>* paragraph [0058] - paragraph [0066] *<br>* figure 4 *<br>----- | 1-15 | |
| A | JOÃ O L CAVALCANTE ET AL: "Aortic Stiffness",<br>JOURNAL OF THE AMERICAN COLLEGE OF CARDIOLOGY, ELSEVIER, AMSTERDAM, NL, vol. 57, no. 14,<br>8 December 2010 (2010-12-08), pages 1511-1522, XP028187753,<br>ISSN: 0735-1097, DOI: 10.1016/J.JACC.2010.12.017<br>[retrieved on 2011-02-21]<br>* abstract *<br>* section "How to Measure AS" *<br>----- | 1-15 | **TECHNICAL FIELDS SEARCHED (IPC)**<br><br>A61B |

The present search report has been drawn up for all claims

| Place of search | Date of completion of the search | Examiner |
|---|---|---|
| Munich | 13 December 2024 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                  

& : member of the same patent family, corresponding document

EPO FORM 1503 03.82 (P04C01)

**EP 4 681 646 A1**

## ANNEX TO THE EUROPEAN SEARCH REPORT
## ON EUROPEAN PATENT APPLICATION NO.

EP 24 30 6190

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2024

| Patent document cited in search report | | Publication date | Patent family member(s) | | Publication date |
|---|---|---|---|---|---|
| US 2017132788 | A1 | 11-05-2017 | CN 108471970 A | | 31-08-2018 |
| | | | DE 112016005178 T5 | | 02-08-2018 |
| | | | US 2017132788 A1 | | 11-05-2017 |
| | | | WO 2017083343 A1 | | 18-05-2017 |
| EP 3832662 | A1 | 09-06-2021 | EP 3832662 A1 | | 09-06-2021 |
| | | | WO 2021110612 A1 | | 10-06-2021 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012107857 A1

- US 8157742 B

**Non-patent literature cited in the description**

- **PEREIRA V. M et al.** A DSA-based Method Using Contrast-Motion Estimation for the Assessment of the Intra-Aneurysmal Flow Changes Induced by Flow-Diverter Stents. *American Journal of Neuroradiology*, 2013, vol. 34 (4), 808-815